# EUROPEAN PATENT APPLICATION

(11) **EP 4 167 353 A1**
(43) Date of publication of application: **19.04.2023**
(21) Application number: 21821451.8
(22) Date of filing: 17.03.2021
(51) Int. Cl.: H01M 14/00, G01F 23/24, G01N 27/02, G01N 27/416

(54) **SENSOR**

(30) Priority: 12.06.2020 JP 2020102687
(71) Applicant: Tripod Design Co., Ltd., Tokyo, 102-0073 (JP)
(72) Inventor: NAKAGAWA, Satoshi, Tokyo 102-0073 (JP)
(74) Representative: Hoffmann Eitle
(86) International application number: PCT/JP2021/010940
(87) International publication number: WO 2021/250970

(57) **Abstract**

An object is to provide a sensor capable of operating on a self-sustaining power source.

A sensor comprising a system, the system including: a first conductive part and a second conductive part; a medium; and a functional part, the first conductive part and the functional part being connected to each other, the second conductive part and the functional part being connected to each other, at least a part of the first conductive part and the second conductive part being in contact with the medium, and the first conductive part and the second conductive part being not in contact with each other, and the sensor being configured to detect that internal impedance of the system satisfies a predetermined condition.

## Description

### Technical Field

The present invention relates to a sensor capable of operating on a self-sustaining power source.

### Background Art

There is a device in which two different kinds of metals are used as electrodes and immersed in an electrolytic solution to cause an oxidation reaction or a reduction reaction in each electrode to create a path through which electrons flow (that is, a battery), such as a voltaic battery or a Daniels battery.

A liquid used as an electrolytic solution of a battery may adversely affect a human body and an environment, such as fear of chemical burn, fear of fire or explosion, or generation of toxic gas, and thus handling of the electrolytic solution is very difficult. In addition, there is a problem in that it is necessary to prevent liquid leakage at the time of commercialization, and the cost increases.

Meanwhile, in recent years, energy harvesting technology that converts minute energy such as visible light, heat, radio waves, or organic matter decomposition processing by microorganisms into electric power has attracted attention, and research has been conducted to put a self-sustaining power source into practical use by using the energy harvesting technology. Furthermore, in the IoT field, research is in progress as a means that makes it possible to obtain various information in various environments by combining a sensor and a self-sustaining power source.

For a self-sustaining power source type device used for a sensor or the like, it has been studied to use solar power generation (see, for example, Patent Literature 1), but it is likely to be affected by a time zone and weather, and stable power supply may not be possible.

### Citation List

### Patent Literature

Patent Literature 1: JP 2007-181278 A

### Summary of Invention

### Technical Problem

At least one object of the present invention is to provide a sensor capable of operating on a self-sustaining power source.

### Solution to Problem

The present invention solves the above problem by any of the following [1] to [14].
[1] A sensor comprising a system, the system including: a first conductive part and a second conductive part; a medium; and a functional part, the first conductive part and the functional part being connected to each other, the second conductive part and the functional part being connected to each other, at least a part of the first conductive part and the second conductive part being in contact with the medium, and the first conductive part and the second conductive part being not in contact with each other, and the sensor being configured to detect that internal impedance of the system satisfies a predetermined condition;
[2] A sensor comprising a system, the system including: a first conductive part and a second conductive part; a medium; and a functional part, the first conductive part and the functional part being connected to each other, the second conductive part and the functional part being connected to each other, at least a part of the first conductive part and the second conductive part being in contact with the medium, and the first conductive part and the second conductive part being not in contact with each other, and the sensor being configured to detect that a predetermined voltage in the system satisfies a predetermined condition;
[3] A sensor comprising a system, the system including: a first conductive part and a second conductive part; a medium; and a functional part, the first conductive part and the functional part being connected to each other, the second conductive part and the functional part being connected to each other, at least a part of the first conductive part and the second conductive part being in contact with the medium, and the first conductive part and the second conductive part being not in contact with each other, and the sensor being configured to detect that a change amount per unit time of internal impedance of the system satisfies a predetermined condition;
[4] A sensor comprising a system, the system including: a first conductive part and a second conductive part; a medium; and a functional part, the first conductive part and the functional part being connected to each other, the second conductive part and the functional part being connected to each other, at least a part of the first conductive part and the second conductive part being in contact with the medium, and the first conductive part and the second conductive part being not in contact with each other, and the sensor being configured to detect that a change amount per unit time of a predetermined voltage in the system satisfies a predetermined condition;
[5] The sensor according to any one of [1] to [4], wherein the functional part includes a voltage boost circuit or a voltage step-down circuit;
[6] The sensor according to [2] or [4], wherein the functional part includes a voltage boost circuit or a voltage step-down circuit, and the predetermined voltage in the system is an input voltage or an output voltage of the voltage boost circuit or the voltage step-down circuit;
[7] The sensor according to any one of [1] to [6], wherein the functional part has a conversion function of converting output impedance;
[8] The sensor according to any one of [1] to [7], wherein the functional part includes an electric storage part, and the electric storage part stores electric charge supplied from the first conductive part and/or the second conductive part, and the electric storage part has a function of releasing stored electric charge in a time shorter than a time required for storing;
[9] The sensor according to any one of [1] to [8], wherein the functional part includes an output voltage conversion part;
[10] The sensor according to any one of [1] to [9], wherein input impedance of the functional part has a non-linear current-voltage characteristic;
[11] A device comprising: a first conductive part and a second conductive part; and a functional part, the first conductive part and the functional part being connected to each other, the second conductive part and the functional part being connected to each other, and the first conductive part and the second conductive part being not in contact with each other, and the device being configured to detect that internal impedance of a system satisfies a predetermined condition, the system being configured in a case where the first conductive part and the second conductive part are brought into contact with a medium;
[12] A device comprising: a first conductive part and a second conductive part; and a functional part, the first conductive part and the functional part being connected to each other, the second conductive part and the functional part being connected to each other, and the first conductive part and the second conductive part being not in contact with each other, and the device being configured to detect that a predetermined voltage in a system satisfies a predetermined condition, the system being configured in a case where the first conductive part and the second conductive part are brought into contact with a medium;
[13] A device comprising: a first conductive part and a second conductive part; and a functional part, the first conductive part and the functional part being connected to each other, the second conductive part and the functional part being connected to each other, and the first conductive part and the second conductive part being not in contact with each other, and the device being configured to detect that a change amount per unit time of internal impedance of a system satisfies a predetermined condition, the system being configured in a case where the first conductive part and the second conductive part are brought into contact with a medium;
[14] A device comprising: a first conductive part and a second conductive part; and a functional part, the first conductive part and the functional part being connected to each other, the second conductive part and the functional part being connected to each other, and the first conductive part and the second conductive part being not in contact with each other, and the device being configured to detect that a change amount per unit time of a predetermined voltage in a system satisfies a predetermined condition, the system being configured in a case where the first conductive part and the second conductive part are brought into contact with a medium.

### Advantageous Effects of Invention

According to the present invention, it is possible to provide a sensor capable of operating on a self-sustaining power supply.

### Brief Description of Drawings

Fig. 1 is a block diagram illustrating a configuration of a system according to an embodiment of the present invention.
Fig. 2 is a block diagram illustrating a configuration of an electric power conversion part according to an embodiment of the present invention.
Fig. 3 is a diagram illustrating a system according to an embodiment of the present invention.
Fig. 4 is a diagram illustrating a relationship between time and a current I in a case where ON/OFF of a transistor in the system is switched according to an embodiment of the present invention.

### Description of Embodiments

Hereinafter, embodiments of the present invention will be described with reference to the accompanying drawings. Hereinafter, the description regarding effects is an aspect of the effects of the embodiments of the present invention, and is not limited to the description herein.

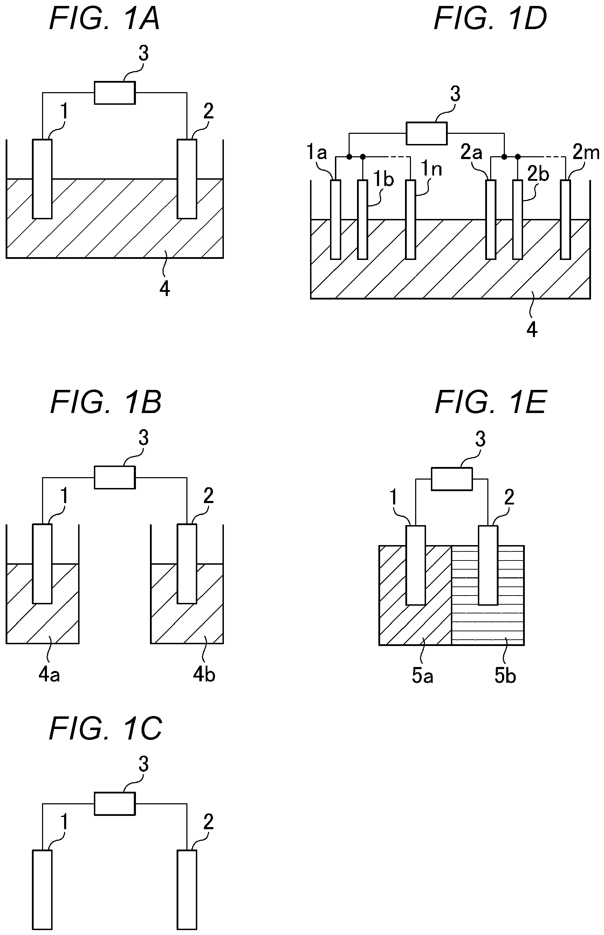

Fig. 1 is a block diagram illustrating a configuration of a system according to an embodiment of the present invention. As shown in Figs. 1A to 1E, the system includes a first conductive part 1, a second conductive part 2, a functional part 3, and a medium 4. The first conductive part 1 and the functional part 3 are electrically connected to each other, and the functional part 3 and the second conductive part 2 are electrically connected to each other. The term "electrically connected" means, for example, energizably connected by a conductive wire or the like.

A part or the whole of the first conductive part 1 and the second conductive part 2 are in contact with the medium 4. The first conductive part 1 and the second conductive part 2 are not in contact with each other. The term "not in contact" refers to, for example, a state in which the first conductive part 1 and the second conductive part 2 are not in direct contact with each other.

The distance between the position where the first conductive part 1 is in contact with the medium 4 and the position where the second conductive part 2 is in contact with the medium 4 is preferably 1 cm or more, more preferably 10 cm or more, still more preferably 100 cm or more, and particularly preferably 1000 cm (10 m) or more.

It is preferable that both the first conductive part 1 and the second conductive part 2 have conductivity. Here, examples of the material of the first conductive part 1 and the second conductive part 2 include metal, a conductive polymer, carbon, and the like. Shapes of the first conductive part 1 and the second conductive part 2 are not particularly limited. The first conductive part 1 and the second conductive part 2 may have a rectangular parallelepiped shape, a round columnar shape (rod shape), a pyramid shape, a conical shape, a plate shape, or a string shape, and may have any shape.

The metal used for the first conductive part 1 and the second conductive part 2 can be appropriately selected from, for example, silver, copper, gold, aluminum, magnesium, zinc, nickel, platinum, tin, titanium, stainless steel, zinc oxide, magnesium oxide, oxides of the above-described metals, and the like. In addition, a predetermined metal may be coated with another metal different from the predetermined metal or another material having conductivity.

Materials of the first conductive part 1 and the second conductive part 2 may be different from each other, or may be the same as each other. For example, a round columnar rod made of stainless steel can be used for the first conductive part 1, and a round columnar rod made of zinc can be used for the second conductive part 2. In this case, the first conductive part 1 and the second conductive part 2 are connected to the functional part 3 or a voltage boost circuit/voltage step-down circuit by a conductive wire.

When polarization resistance is measured for at least one of the first conductive part 1 or the second conductive part 2 using an AC impedance method, the measured value is preferably 100 Q or more.

Here, a conductive part serving as a starting point of a current is defined as the first conductive part 1, and a conductive part serving as an ending point is defined as the second conductive part 2. Which conductive part functions as the first conductive part 1 is determined by a material of the conductive part or an environment surrounding the conductive part (for example, temperature, humidity, atmospheric pressure, pH, and the like). A chemical reaction occurs at an interface between the first conductive part 1 or the second conductive part 2 and the medium 4, and free electrons are generated in the conductive part.

For example, when different metals are used for the first conductive part 1 and the second conductive part 2, the conductive part made of a metal having a lower standard electrode potential is used as the first conductive part 1, and the conductive part made of a metal having a higher standard electrode potential is used as the second conductive part 2. In this case, electrons move from the second conductive part 2 toward the functional part 3, and electrons move from the functional part 3 toward the first conductive part 1. That is, a current is generated from the first conductive part 1 side to the second conductive part 2 side via the functional part 3. For example, in the second conductive part 2, the metal constituting the conductive part is eluted as a cation into the medium 4 to generate free electrons, and in the first conductive part 1, the cation in water of the medium 4 reacts with the electrons to be electrically neutralized.

The level of the standard electrode potential is determined by comparing relative values (relative values) of the standard electrode potentials of substances, and is not determined by using an absolute value of the standard electrode potential. For example, when substance A having a standard electrode potential of -5 V is compared with substance B having a standard electrode potential of +2 V, the standard electrode potential of substance A is low, and the standard electrode potential of substance B is high.

On the other hand, even when the same metal is used for the conductive parts, either one of the conductive parts functions as the first conductive part 1 and the other conductive part functions as the second conductive part 2 depending on the conditions of the surrounding environment of the conductive parts, such as temperature, humidity, atmospheric pressure, and pH, for example, and a current is generated. Therefore, when conditions such as ambient temperature, humidity, atmospheric pressure, and pH of the two conductive parts are changed, the one functioning as the first conductive part may function as the second conductive part, and the one functioning as the second conductive part may function as the first conductive part.

The electromotive force generated from the first conductive part 1 and the second conductive part 2 is preferably 0.9 V or less, more preferably 0.35 V or less, and still more preferably 0.25 V or less. The electromotive force generated from the first conductive part 1 and the second conductive part 2 is preferably 5 mV or more.

The functional part 3 is, for example, a part that executes a predetermined function by energization. The functional part 3 can include an electric consumption part that consumes electric power and exerts a predetermined function, an electric storage part that stores electricity generated in a conductive part, an output voltage conversion part or the like that converts a voltage to be output, such as a voltage boost circuit and a voltage step-down circuit, a control part such as a microcomputer that controls a circuit, and a communication part or the like capable of wirelessly communicating with other devices.

As the electric consumption part, for example, any of a light source such as an incandescent light bulb or a light emitting diode, a heat generator that emits heat, a sounding body that emits sound, a transmitter that emits a signal, and the like can be adopted. The electric storage part may be included in the voltage boost circuit or the voltage step-down circuit. A control part such as a microcomputer can control a circuit to release the electricity stored in the electric storage part under a predetermined condition. The released electricity is consumed by the electric consumption part. In addition, even in the control part such as a microcomputer, electric power is consumed slightly, and thus, it is possible to perform control so as to release the stored electricity while securing electric power necessary for activating the control part.

The functional part 3 may include any one of the electric consumption part, the electric storage part, the output voltage conversion part, the communication part, and the control part, and may be formed by combining any two or more of the power consumption part, the electric storage part, the output voltage conversion part, the communication part, and the control part. In addition, the functional part 3 may be formed by integrally forming any two or more of the electric consumption part, the electric storage part, the output voltage conversion part, the communication part, and the control part, or may be formed separately while electrically connecting any of the electric consumption part, the electric storage part, the output voltage conversion part, the communication part, and the control part.

Input impedance in the functional part 3 is preferably 1 kΩ or more, and more preferably 10 kΩ or more. In addition, the input impedance of the functional part 3 preferably has a non-linear current-voltage characteristic (I-V characteristic). The non-linear current-voltage characteristic refers to, for example, a case where, in a voltage change when a current flows through the functional part 3, the voltage value increases as a current value increases, but as the current value increases, an increase width of the voltage value required to increase the current value increases, and the voltage is not proportional to the current. In other words, the current value increases as the voltage value applied to the functional part 3 increases, but the degree of increase in the current value, which is increased by an increase in the voltage value, decreases as the voltage value increases, and the current value is not proportional to the voltage value. Since the input impedance in the functional part 3 has the non-linear current-voltage characteristic, the electromotive force generated between the first conductive part 1 and the second conductive part 2 is easily maintained.

The functional part 3 preferably has a function of converting output impedance. As a result, an influence on an input signal of the functional part 3 can be controlled.

In addition, the functional part 3 includes the electric storage part, and stores electric charge supplied from the first conductive part and/or the second conductive part. The control part performs control to release the stored electric charge in a time shorter than a time required to store the electric charge.

The lower limit value of the operating voltage of the functional part 3 is preferably 0.9 V or less. It is more preferable to operate at 0.35 V or less, and it is still more preferable to operate at 20 mV or less.

The medium 4 may be in any form of a gas, a liquid, and a solid. The medium 4 may be sol or gel. The medium 4 is not particularly limited as long as it can cause a chemical reaction at the interface between the first conductive part 1 and the second conductive part 2. The medium 4 may contain an electrolyte. As the medium 4, a medium having no conductivity is preferably used. The gas is not particularly limited as long as it is a gas when used in the present system, and examples thereof include oxygen, carbon dioxide, nitrogen, hydrogen, methane, and the like. When a gas is used as the medium 4, only a single type of gas may be used, or a mixture of a plurality of types of these gases may be used.

The liquid used as the medium 4 is not particularly limited as long as it is a liquid when used in the present system, and, for example, not only water but also an organic solvent having high polarity, an organic solvent having low polarity, or a non-polar organic solvent can be used. The liquid used as the medium 4 may be a mixture of water and an organic solvent having high polarity, a mixture of two or more different organic solvents, an emulsion, or the like. As the water, not only pure water but also water containing an electrolyte can be used.

Among electrolytes contained in the water, the concentration of cations may be 1 mol/L or less, 0.6 mol/L or less, 0.1 mol/L or less, 0.01 mol/L or less, 0.001 mol/L or less, or 0.0001 mol/L or less.

As the organic solvent having high polarity, for example, lower alcohols such as methanol and ethanol, lower carboxylic acids such as formic acid and acetic acid, acetone, tetrahydrofuran, dimethyl sulfoxide, and the like can be used. As the organic solvent having low polarity, higher alcohols such as hexanol and octanol, higher carboxylic acids such as hexanoic acid and octanoic acid, or the like can be used. Examples of the non-polar organic solvent include aliphatic hydrocarbons such as hexane, octane, and nonane, and aromatic compounds such as benzene, toluene, and xylene. When a liquid is used as the medium 4, only a single type of liquid may be used, or a mixture of a plurality of types of these liquids may be used.

The solid used as the medium 4 is not particularly limited as long as it is a solid when used in the present system, and may be, for example, wood, plastic, metal, ceramics, concrete, or the like. As the solid used as the medium 4, for example, a powdery or granular solid such as sand or soil can be used, and a plurality of stones or rocks stacked can also be used. When sand, soil, stones, or rocks stacked on one another are used as the medium 4, fine voids are generated in the medium 4, but even if there are such voids, it is sufficient that the medium 4 is physically connected.

One medium 4 can be formed by physically connecting media 4 having different compositions and characteristics. For example, when plastic is in contact with the first conductive part 1 and wood is in contact with the second conductive part 2, the plastic and wood come into contact with each other to function as the medium 4.

Resistance value of the medium 4 between the first conductive part 1 and the second conductive part 2 is preferably 1 kΩ or more, and more preferably 10 kΩ or more.

The medium 4 may be an insulator. The insulator refers to, for example, a non-conductor, and refers to a substance having a property of hardly passing electricity or heat. The electrical conductivity of the insulator is preferably 10⁻⁵ S/m or less, more preferably 10⁻⁸ S/m, and still more preferably 10⁻¹¹ S/m.

The medium 4 preferably contains moisture. The water content of the medium 4 such as sand or soil is preferably 1% by mass or more, more preferably 5% by mass or more, and still more preferably 10% by mass or more. The water content of the medium 4 is preferably 200% by mass or less. Here, the water content refers to a value obtained by dividing the weight of water by the sum of the weight of water and the weight of solid content.

As illustrated in Fig. 1A, a system according to an embodiment of the present invention includes the first conductive part 1, the second conductive part 2, the functional part 3, and the medium 4. The medium 4 is in contact with both the first conductive part 1 and the second conductive part, and the functional part 3 is electrically connected to the first conductive part 1 and the second conductive part 2.

As illustrated in Fig. 1B, a system according to an embodiment of the present invention may use a medium 4a and a medium 4b instead of the medium 4. The medium 4a and the medium 4b may be different types of media from each other, or may be the same type of media. In Fig. 1B, the medium 4a and the medium 4b are housed in different containers and are in a not-in-contact state.

As illustrated in Fig. 1C, a system according to an embodiment of the present invention may electrically connect the first conductive part 1 and the second conductive part 2 without using the medium 4.

As illustrated in Fig. 1D, in a system according to an embodiment of the present invention, a plurality of first conductive parts 1a, 1b to 1n (n is an integer of 2 or more) may be electrically connected in parallel. In addition, a plurality of second conductive parts 2a, 2b to 2m (m is an integer of 2 or more) may be electrically connected in parallel. Note that the plurality of first conductive parts 1a, 1b to 1n may be electrically connected in series. Furthermore, the plurality of second conductive parts 2a, 2b to 2m may be electrically connected in series.

As illustrated in Fig. 1E, in at least one embodiment of the present invention, a part or the whole of the first conductive part 1 may be covered with an insulating part 5a, and a part or the whole of the second conductive part 2 may be covered with an insulating part 5b. By bringing the insulating parts 5a and 5b into contact with each other, the insulating parts 5a and 5b function as the medium 4. Here, the insulating parts 5a and 5b may be made of insulators different from each other, or may be made of insulators having the same composition or properties.

The configurations illustrated in Figs. 1A to 1E may be combined. The combination is not limited as long as it is an aspect that exerts the effect of the present invention.

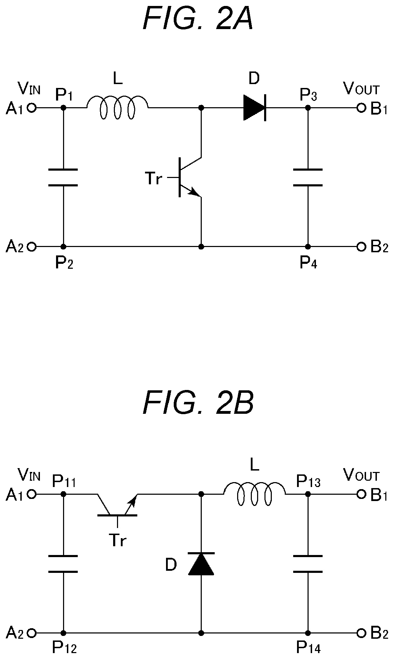

Fig. 2 is a block diagram illustrating a configuration of an electric power conversion part according to an embodiment of the present invention. Fig. 2A is a circuit diagram of a voltage boost circuit according to an embodiment of the present invention. A voltage boost circuit or a voltage step-down circuit is an example of the functional part 3, and includes an electric storage part.

As illustrated, an inductor L, a diode D, a transistor Tr, and a capacitor C are electrically connected. For example, an input terminal A1 is connected to the first conductive part 1, and an input terminal A2 is connected to the second conductive part 2. An output terminal B1 and an output terminal B2 are connected to an electric consumption part, a control part, and the like. The control part may be connected in parallel with the voltage boost circuit between the voltage boost circuit, and the first conductive part 1 and the second conductive part 2.

When an input voltage V_{IN} is applied while the transistor Tr is ON, electric energy is stored in the inductor L. The input voltage V_{IN} is the potential difference between a connection point P₁ and a connection point P₂. When the transistor Tr is OFF, the energy stored in the inductor L is added to the electric energy derived from the input voltage V_{IN}, and is output via the diode D. As a result, an output voltage V_{OUT}, which is the potential difference between a connection point P₃ and a connection point P₄, is higher than the input voltage V_{IN}. The voltage boost circuit may be based on the premise that the input voltage V_{IN} is a voltage lower than a predetermined voltage, and boost control may not be executed at a voltage higher than the predetermined voltage. The input voltage V_{IN} of the voltage boost circuit is preferably 5 mV or more. Note that ON/OFF of the transistor Tr is controlled by the control part.

Fig. 2B is a circuit diagram of a voltage step-down circuit according to an embodiment of the present invention. As illustrated, the transistor Tr, the inductor L, the diode D, and the capacitor C are electrically connected. For example, the input terminal A1 is connected to the first conductive part 1, and the input terminal A2 is connected to the second conductive part 2. The output terminal B1 and the output terminal B2 are connected to the electric consumption part, the control part, and the like. The control part may be connected in parallel with the voltage step-down circuit between the voltage step-down circuit, and the first conductive part 1 and the second conductive part 2.

When the transistor Tr is ON, electric energy is stored in the inductor L. The input voltage V_{IN} is the potential difference between a connection point P₁₁ and a connection point P₁₂, and the output voltage V_{OUT} is the potential difference between a connection point P₁₃ and a connection point P₁₄. In this case, the input voltage V_{IN} is substantially equal to the output voltage V_{OUT}. When the transistor Tr is OFF, the potential of at a connection point P₁₅ at the left end of the inductor L becomes lower than the potential at the connection point P₁₄, so that the output voltage V_{OUT} becomes a lower voltage. The voltage step-down circuit may be based on the premise that the input voltage V_{IN} is higher than a predetermined voltage, and step-down control may not be executed at a voltage lower than the predetermined voltage. Note that ON/OFF of the transistor Tr is controlled by the control part.

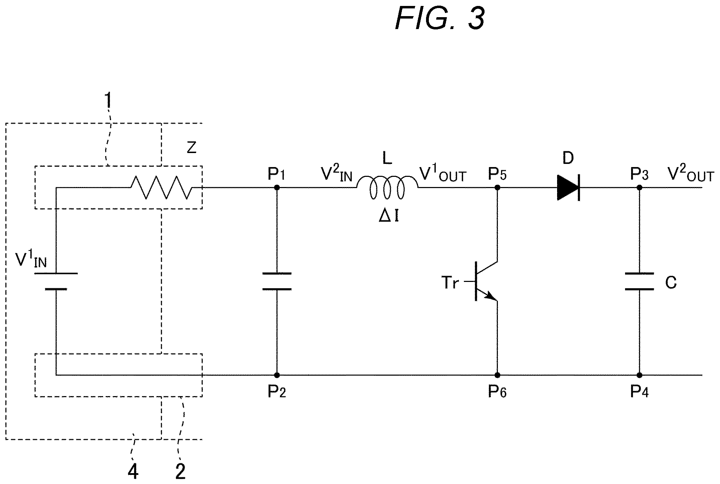

Next, a method for measuring the internal impedance of a system of the present invention will be described. Fig. 3 is a diagram illustrating a system according to an embodiment of the present invention. The potential difference between the first conductive part 1 and the second conductive part 2 can be defined as V¹_{IN}, and the potential difference between the connection point P₁ and the connection point P₂ can be defined as V²_{IN}. The potential difference between a connection point P₅ and a connection point P₆ can be defined as V¹_{OUT}, and the potential difference between the connection point P₃ and the connection point P₄ can be defined as V²_{OUT}. When power is generated by the system of the present invention, a current I flows between the first conductive part 1 and the second conductive part 2 in the direction of the connection point P₁ and the connection point P₅ by electromotive force V²_{IN}.

As illustrated in Fig. 3, a voltage boost circuit is connected to the first conductive part 1 at the connection point P₁, and to the second conductive part 2 at the connection point P₂. In the voltage boost circuit, the inductor L, the diode D, the transistor Tr, and the capacitor C are electrically connected.

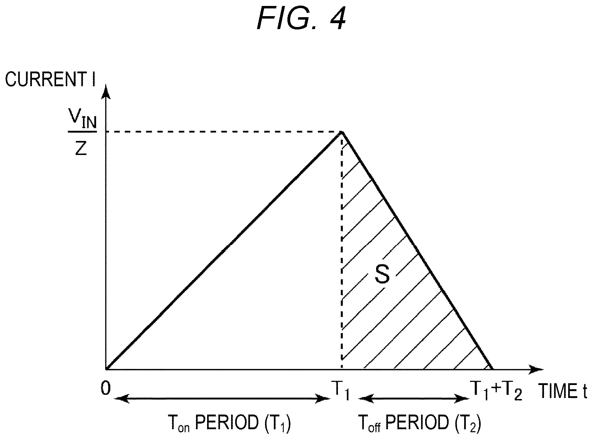

Fig. 4 is a diagram illustrating a relationship between time and the current I when ON/OFF of the transistor in the system is switched according to an embodiment of the present invention. Here, the relationship between V¹_{OUT} and V²_{IN} can be expressed by Equation (1): V¹_{OUT} - V²_{IN} = -L1 × dI/dt using the current I flowing through the inductor L and inductance L1. When the transistor Tr is ON, since V²_{OUT} = 0, Equation (2): V²_{IN} = L1 × dI/dt can be derived. In this case, dI/dt is a positive value, and the current I increases with time. On the other hand, when the transistor Tr is OFF, since V¹_{OUT} > V²_{IN} is satisfied, it can be seen from Equation (1): V¹_{OUT} - V²_{IN} = -L1 × dI/dt that dI/dt is a negative value. In this case, the current I decreases with time. The ON and OFF of the transistor Tr are periodically repeated.

Here, when the first conductive part 1, the second conductive part 2, and the medium 4 are regarded as one type of battery, it can be considered that the current I flows due to the electromotive force V¹_{IN}. In this case, when the internal impedance caused by the medium 4 is defined as Z, the relationship between an input voltage and the internal impedance can be expressed by Equation (3): V¹_{IN} = Z × I + V²_{IN}.

In addition, while the transistor Tr is OFF (hereinafter, referred to as a T_{OFF} period), the capacitor C is charged with electric charge Q by the current I. Assuming that the voltage increased at the connection point P₃ during the T_{OFF} period is ΔV and the capacitor capacitance of the capacitor C is C1, Equation (4) : Q = ∫Idt = C1 × ΔV holds.

From Equations (2) and (3), V¹_{IN} = L1 × dI/dt + Z × I is derived. By solving this equation, Equation (5): I(t) = V¹_{IN}/Z + A × e^{(-Z/L1 × t)} is derived, where A is an integral constant. In a case where the time when the transistor Tr is switched from OFF to ON is t = 0, as is clear from Fig. 4, the current I is zero when t = 0. Therefore, when t = 0 and I = 0 are substituted into Equation (5), it is found that the relationship of A = -V¹_{IN}/Z holds. When this A = -V¹_{IN}/Z is substituted into Equation (5), Equation (6): I(t) = V¹_{IN}/Z × (1 - e(^{-Z/L1 × t)}) can be derived. The current I while the transistor Tr is ON (hereinafter, referred to as a Tₒₙ period) can be calculated by Equation (6). When a time during which the transistor Tr is ON is sufficiently taken, the maximum value of the current I is V¹_{IN}/Z.

The current I when the Tₒₙ period ends and the T_{off} period starts (that is, when time T1 has elapsed since the transistor Tr was switched from OFF to ON) can be calculated by substituting t = T1 into Equation (6). This is because the current I has continuity as can be seen from Fig. 4. When replacing (1 - e(^{-Z/L1 × T1})) with K (constant), the current I at t = T1 can be expressed as I(T1) = V¹_{IN}/Z × (1 - e^{(-Z/L1 × T1)}) = K × V¹_{IN}/Z. Note that K satisfies the relationship of 0 ≤ K < 1, and when the value of Z/L1 × T1 becomes sufficiently large, K can be approximated to 1.

Next, Equation (7) : L1 × dI/dt + Z × I = V¹_{IN} - V¹_{OUT} can be derived by Equations (1) and (3). Furthermore, by Equation (4), V²_{OUT} can be expressed by fIdt/C1 + Vₛₜₐᵣₜ. Here, Vₛₜₐᵣₜ is the voltage of the capacitor C at the start of the T_{off} period (t = T1) and is a constant. If the threshold voltage of the diode D is V_{f}, Equation (8) : V¹_{OUT} = V²_{OUT} + V_{f} = ∫Idt/C1 + Vₛₜₐᵣₜ + V_{f} = ∫Idt/C1 + V'ₒᵤₜ can be derived. Here, V'ₒᵤₜ = Vₛₜₐᵣₜ + V_{f} is a constant.

Furthermore, Equation (9): fIdt/C1 + Z × I + L1 × dI/dt = V¹_{IN} - V'ₒᵤₜ can be derived from Equations (7) and (8). By solving the differential equation of Equation (9), the current I during the T_{off} period can be expressed by a function of time t, the capacitor capacitance C1, the internal impedance Z, the inductance L1, V¹_{IN}, V'ₒᵤₜ, and K. When the start time of the T_{off} period is t = 0, an initial value I (0) of the current I at that time is 1(0) = K × V¹_{IN}/Z. When the T_{off} period ends (that is, time T2 elapses after the transistor Tr is switched from ON to OFF, and the current I becomes zero), I(T2) = 0. The capacitor capacitance C1, the inductance L1, and V'ₒᵤₜ are constants, and when I(0) and T2 are measured, the values of V¹_{IN} and Z can be calculated.

Unlike the method described above, Z can be easily obtained. During the T_{off} period, since V¹_{OUT} is a voltage that is about 10 times larger than V²_{IN}, dI/dt also has a large value. In this case, fIdt in Equation (4) corresponds to the area of the triangle S in Fig. 4. Therefore, Equation (9): fIdt = K × V¹_{IN}/Z × T2/2 = C1 × ΔV is derived from Equation (4). Here, if the Tₒₙ time is sufficiently long, K ≈ 1 can be approximated, and thus, when K = 1 is substituted into Equation (9), Equation (10): V¹_{IN}/Z × T2/2 = C1 × ΔV is derived. C1 is a constant, and Z can be calculated from ΔV at the time point when the T_{off} period is sufficiently long (the time point when the current I becomes the minimum value), V¹_{IN} at the time point when the T_{off} period is sufficiently long (the time point when the current I becomes the minimum value), and T2 (the time when V¹_{OUT} becomes equal to V²_{IN}). Since V¹_{IN} = V²_{IN} at the time point when the T_{off} period is sufficiently long (the time point when the current I is consumed), V¹_{IN} can be specified by measuring V²_{IN}.

Note that the calculation of the internal impedance Z is executed by the control part.

### (First embodiment of sensor)

A sensor according to a first embodiment of the present invention is a sensor utilizing the above-described system. Examples thereof include a sensor that detects whether the internal impedance Z, or the input voltage V_{IN} or the output voltage V_{OUT} in the voltage boost circuit or the voltage step-down circuit in the above system satisfies a predetermined condition. Here, the predetermined condition can be appropriately designed such that the internal impedance Z, the input voltage V_{IN}, or the output voltage V_{OUT} exceeds a predetermined threshold, is equal to or greater than a predetermined threshold, is less than a predetermined threshold, or is equal to or less than a predetermined threshold. The input voltage V_{IN} to be a target of the predetermined condition may be, for example, the input voltage V²_{IN} when the T_{off} period is sufficiently long and the current I becomes zero, and the output voltage V_{OUT} to be a target of the predetermined condition may be, for example, the output voltage V²_{OUT} when the T_{off} period is sufficiently long and the current I becomes zero. Furthermore, the input voltage V_{IN} to be a target of the predetermined condition may be, for example, the input voltage V²_{IN} at the time when the T_{off} period is started, and the output voltage V_{OUT} to be a target of the predetermined condition may be, for example, the output voltage V²_{OUT} at the time when the T_{off} period is started. The input voltage V²_{IN} at the start of the T_{off} period is a value dependent on the impedance Z as is clear from Equation (3). Conversely, by measuring the input voltage V²_{IN}, the fluctuation of the input voltage including the impedance Z can be grasped. Therefore, the input voltage V²_{IN} at the start of the T_{off} period is effective as the measurement data. When the internal impedance Z, the input voltage V_{IN}, or the output voltage V_{OUT} satisfies a predetermined condition, a signal is transmitted from the communication part to another computer device under the control of the control part.

For example, the sensor of the present invention can be used using water such as of a river, a pond, a sea, or a dam as a medium. The sensor is installed such that the longitudinal directions of the first conductive part 1 and the second conductive part 2 of the sensor are perpendicular to the water surface. When the internal impedance, or the input voltage V_{IN} or the output voltage V_{OUT} in the system satisfies a predetermined condition due to a change in the height of the water surface, a signal is transmitted to another computer device. When the signal is received by the other computer device, information indicating that the height of the water surface exceeds a threshold or the like is notified is displayed on a display screen of the other computer device or output as a voice by the other computer device. As described above, by using the sensor of the present invention, it is possible to grasp, by another computer device that the height of the water surface becomes high and danger is imminent around the installation position of the sensor.

In the first embodiment, the change in the height of the water surface is detected by the sensor, however, for example, even when there is a change in the water quality (pH or composition) at the installation position of the sensor, this change can be detected based on the internal impedance, or the input voltage V_{IN} or the output voltage V_{OUT} in the system. In addition, in the first embodiment, it has been described that water such as of a river, a pond, a sea, or a dam is used as a medium, but the sensor of the present invention can use, for example, soil or the like different from water as a medium.

### (Second embodiment of sensor)

A sensor according to a second embodiment of the present invention is a sensor utilizing the above-described system. Examples thereof include a sensor that detects that a change amount per unit time of the internal impedance, or the input voltage V_{IN} or the output voltage V_{OUT} in the voltage boost circuit or the voltage step-down circuit in the above system satisfies a predetermined condition. Here, the predetermined condition can be appropriately designed such that the change amount per unit time of the internal impedance, or the input voltage V_{IN} or the output voltage V_{OUT} exceeds a predetermined threshold, is equal to or greater than a predetermined threshold, is less than a predetermined threshold, or is equal to or less than a predetermined threshold. When the change amount per unit time of the internal impedance, or the input voltage V_{IN} or the output voltage V_{OUT} satisfies a predetermined condition, a signal is transmitted from the communication part to another computer device under the control of the control part.

For example, the sensor of the present invention can be used using water such as of a river, a pond, a sea, or a dam as a medium. The sensor is installed such that the longitudinal directions of the first conductive part 1 and the second conductive part 2 of the sensor are perpendicular to the water surface. When the change amount per unit time of the internal impedance, or the input voltage V_{IN} or the output voltage V_{OUT} in the system satisfies a predetermined condition due to the change in the height of the water surface, a signal is transmitted to another computer device. When the signal is received by the other computer device, information indicating that the height of the water surface suddenly rises (alternatively, the height of the water surface has fallen sharply) or the like is notified is displayed on a display screen of the other computer device or output as a voice by the other computer device. As described above, by using the sensor of the present invention, it is possible to grasp, by another computer device that the height of the water surface suddenly rises and danger is imminent around the installation position of the sensor.

In the second embodiment, it has been described that water such as of a river, a pond, a sea, or a dam is used as a medium, but the sensor of the present invention can use, for example, soil or the like different from water as a medium.

In the embodiments of the present invention, a "system" refers to, for example, a system including elements necessary for achieving the effects of the present invention. The system more specifically includes a circuit and a device. The "conductive part" may be, for example, made of any material as long as it is an energizable member. The "functional part" is, for example, a part that executes a predetermined function by energization. The function may be to convert electricity into energy such as light or heat, or to control a circuit.

In the embodiments of the present invention, the "electrolytic solution" refers to, for example, a solution having electrical conductivity in which an ionic substance is dissolved in a polar solvent. The "voltage boost circuit" refers to, for example, a circuit that boosts and outputs an input voltage. The "voltage step-down circuit" refers to, for example, a circuit that steps down and outputs an input voltage. The "conductive polymer" refers to, for example, a polymer compound having electrical conductivity. "Carbon" refers to, for example, carbon fiber having conductivity. The "integrally forming" refers to, for example, joining different objects to each other, and more specifically, joining by a chemical and/or physical force such as adhesion using an adhesive, mechanical joining using another member, welding, and pressure bonding can be mentioned.

### Examples

Hereinafter, the present invention will be described in more detail with reference to examples, but the present invention is not limited by these examples at all.

### (Example 1)

The following test was performed at normal temperature and normal pressure. A circuit including the first conductive part 1, the second conductive part 2, the functional part 3, and the medium 4 illustrated in Fig. 1A was used. A plate-shaped member (0.5 mm thick, 10 cm × 15 cm) made of stainless steel (austenite, SUS 304 series) was used as the first conductive part 1, a plate-shaped member (0.5 mm thick, 10 cm × 15 cm) made of a galvanized steel plate (iron) was used as the second conductive part 2, and the first conductive part 1, the second conductive part 2, and the functional part 3 were connected to each other by copper conductive wires. The functional part 3 includes an electric consumption part, an output voltage conversion part, a communication part, and a control part. In addition, the functional part 3 having an input impedance of 1 kΩ or more and a non-linear current-voltage characteristic was used. As the electric consumption part, an LED bulb that lights up when a current of 2 mA or more flows was used. A voltage boost circuit illustrated in Fig. 2A was used as the output voltage conversion part to form a system.

The first conductive part 1 was connected to the input terminal A1 of the voltage boost circuit of the output voltage conversion part, and the output terminal B1 of the voltage boost circuit was connected to the LED bulb. Furthermore, the second conductive part 2 was connected to the input terminal A2 of the voltage boost circuit, and the output terminal B2 of the voltage boost circuit was connected to a terminal opposite to the terminal connected to the output terminal B1 of the LED bulb.

Pure water (KOGA Chemical Mfg Co.,Ltd., high-purity purified water, temperature 25°C: medium 4) was placed in an acrylic container (cubic body with outer diameter of 15 cm × 15 cm × 15 cm, inner diameter of 14.5 cm) up to a height of 7.5 cm, and the first conductive part 1 and the second conductive part 2 were immersed in the pure water to construct the system. The first conductive part 1 and the second conductive part 2 were not in contact with each other, the distance between the first conductive part 1 and the second conductive part 2 was 12 cm, and the first conductive part 1 and the second conductive part 2 were installed so that plate-like planes of the first conductive part 1 and the second conductive part 2 were parallel to each other.

For the constructed system, the voltage between the first conductive part 1 and the second conductive part 2 was measured (Measurement 1). For the measurement, a 34401A multimeter manufactured by Agilent Technologies was used. The result is shown in Table 1. In the system shown in Example 1, the LED bulb repeatedly blinked every 270 to 330 seconds. That is, it was confirmed that electromotive force was generated from the first conductive part 1 and/or the second conductive part 2.

Next, the first conductive part 1 and the second conductive part 2 were immersed, and pure water (KOGA Chemical Mfg Co.,Ltd., high-purity purified water, temperature 25°C: medium 4) was placed in an acrylic container (cubic body with outer diameter of 15 cm × 15 cm × 15 cm, inner diameter of 14.5 cm) up to a height of 7.5 cm, and the first conductive part 1 and the second conductive part 2 were immersed in the pure water. The first conductive part 1 and the second conductive part 2 were not in contact with each other, the distance between the first conductive part 1 and the second conductive part 2 was 12 cm, and the first conductive part 1 and the second conductive part 2 were installed so that plate-like planes of the first conductive part 1 and the second conductive part 2 were parallel to each other. The first conductive part 1 and the second conductive part 2 were not electrically connected. Then, the voltage between the first conductive part 1 and the second conductive part 2 was measured using a 34401A multimeter (Measurement 2). Furthermore, in this state, the resistance value of the medium 4 between the first conductive part 1 and the second conductive part 2 was measured (Measurement 3).

### (Example 2)

Measurements 1 to 3 were performed in the same manner as in Example 1 except that the medium 4 was changed to soil (soil of house plant manufactured by PROTOLEAF, Inc.). The results are shown in Table 1. In the system shown in Example 2, the LED bulb repeatedly blinked at substantially equal intervals every 21 to 23 seconds. That is, it was confirmed that electromotive force was generated from the first conductive part 1 and/or the second conductive part 2.

### (Example 3)

Measurements 1 to 3 were performed in the same manner as in Example 1 except that a waste cloth soaked in an aqueous solution in which 5 g of salt (Coarse Salt produced by Hakata Salt Co., Ltd.) was dissolved in 50 g of pure water (the same as in Example 1) was attached to the surfaces of the first conductive part 1 and the second conductive part 2 in contact with the medium 4, and the medium 4 was changed to sand (silica sand having a particle size peak (weight ratio) of about 0.9 mm manufactured by Toyo Matelan Corporation). The results are shown in Table 1. In the system shown in Example 2, the LED bulb repeatedly blinked every 80 to 100 seconds. That is, it was confirmed that electromotive force was generated from the first conductive part 1 and/or the second conductive part 2.

**[Table 1]**

| | Measurement 1 [mV] | Measurement 2 [mV] | Measurement 3 [kΩ] |
|---|---|---|---|
| Example 1 | 239 | 952 | 20 |
| Example 2 | 291 | 822 | 1,700 |
| Example 3 | 253 | 954 | 250 |

### (Example 4)

Pure water placed in an acrylic container up to a height of 7.5 cm in Example 1 was added up to a height of 10 cm. By adding pure water, it was possible to confirm a change in the internal impedance of the system described above. In addition, by adding pure water, a change in the input voltage V²_{IN} when the T_{off} period started was able to be confirmed. The internal impedance was calculated by the above-described calculation method.

### (Example 5)

Pure water placed in an acrylic container up to a height of 7.5 cm in Example 1 was added up to a height of 10 cm over 5 minutes. It was possible to confirm that the change amount per unit time of the internal impedance of the system described above changed. In addition, it was possible to confirm that the change amount per unit time of the input voltage was changed by adding pure water. The internal impedance was calculated by the above-described calculation method. The input voltage is the input voltage V²_{IN} when the T_{off} period starts.

### Reference Signs List

- 1: First conductive part
- 2: Second conductive part
- 3: Functional part
- 4: Medium
- 5: Insulating part

## Claims

1. A sensor comprising a system, the system including:
a first conductive part and a second conductive part;
a medium; and
a functional part,
the first conductive part and the functional part being connected to each other,
the second conductive part and the functional part being connected to each other,
at least a part of the first conductive part and the second conductive part being in contact with the medium, and
the first conductive part and the second conductive part being not in contact with each other, and
the sensor being configured to detect that internal impedance of the system satisfies a predetermined condition.

2. A sensor comprising a system, the system including:
a first conductive part and a second conductive part;
a medium; and
a functional part,
the first conductive part and the functional part being connected to each other,
the second conductive part and the functional part being connected to each other,
at least a part of the first conductive part and the second conductive part being in contact with the medium, and
the first conductive part and the second conductive part being not in contact with each other, and
the sensor being configured to detect that a predetermined voltage in the system satisfies a predetermined condition.

3. A sensor comprising a system, the system including:
a first conductive part and a second conductive part;
a medium; and
a functional part,
the first conductive part and the functional part being connected to each other,
the second conductive part and the functional part being connected to each other,
at least a part of the first conductive part and the second conductive part being in contact with the medium, and
the first conductive part and the second conductive part being not in contact with each other, and
the sensor being configured to detect that a change amount per unit time of internal impedance of the system satisfies a predetermined condition.

4. A sensor comprising a system, the system including:
a first conductive part and a second conductive part;
a medium; and
a functional part,
the first conductive part and the functional part being connected to each other,
the second conductive part and the functional part being connected to each other,
at least a part of the first conductive part and the second conductive part being in contact with the medium, and
the first conductive part and the second conductive part being not in contact with each other, and
the sensor being configured to detect that a change amount per unit time of a predetermined voltage in the system satisfies a predetermined condition.

5. The sensor according to any one of claims 1 to 4, wherein the functional part includes a voltage boost circuit or a voltage step-down circuit.

6. The sensor according to claim 2 or 4, wherein
the functional part includes a voltage boost circuit or a voltage step-down circuit, and
the predetermined voltage in the system is an input voltage or an output voltage of the voltage boost circuit or the voltage step-down circuit.

7. The sensor according to any one of claims 1 to 6, wherein the functional part has a conversion function of converting output impedance.

8. The sensor according to any one of claims 1 to 7, wherein
the functional part includes an electric storage part, and
the electric storage part stores electric charge supplied from the first conductive part and/or the second conductive part, and
the electric storage part has a function of releasing stored electric charge in a time shorter than a time required for storing.

9. The sensor according to any one of claims 1 to 8, wherein the functional part includes an output voltage conversion part.

10. The sensor according to any one of claims 1 to 9, wherein input impedance of the functional part has a non-linear current-voltage characteristic.

11. A device comprising:
a first conductive part and a second conductive part; and
a functional part,
the first conductive part and the functional part being connected to each other,
the second conductive part and the functional part being connected to each other, and
the first conductive part and the second conductive part being not in contact with each other, and
the device being configured to detect that internal impedance of a system satisfies a predetermined condition, the system being configured in a case where the first conductive part and the second conductive part are brought into contact with a medium.

12. A device comprising:
a first conductive part and a second conductive part; and
a functional part,
the first conductive part and the functional part being connected to each other,
the second conductive part and the functional part being connected to each other, and
the first conductive part and the second conductive part being not in contact with each other, and
the device being configured to detect that a predetermined voltage in a system satisfies a predetermined condition, the system being configured in a case where the first conductive part and the second conductive part are brought into contact with a medium.

13. A device comprising:
a first conductive part and a second conductive part; and
a functional part,
the first conductive part and the functional part being connected to each other,
the second conductive part and the functional part being connected to each other, and
the first conductive part and the second conductive part being not in contact with each other, and
the device being configured to detect that a change amount per unit time of internal impedance of a system satisfies a predetermined condition, the system being configured in a case where the first conductive part and the second conductive part are brought into contact with a medium.

14. A device comprising:
a first conductive part and a second conductive part; and
a functional part,
the first conductive part and the functional part being connected to each other,
the second conductive part and the functional part being connected to each other, and
the first conductive part and the second conductive part being not in contact with each other, and
the device being configured to detect that a change amount per unit time of a predetermined voltage in a system satisfies a predetermined condition, the system being configured in a case where the first conductive part and the second conductive part are brought into contact with a medium.
